(19) Europäisches Patentamt
European Patent Office
Office européen des brevets

(11) Veröffentlichungsnummer: **0 485 927 A1**

(12) **EUROPÄISCHE PATENTANMELDUNG**

(21) Anmeldenummer: **91119184.9**

(22) Anmeldetag: **11.11.91**

(51) Int. Cl.[5]: **C07D 275/06**, C11D 3/395, C07D 275/02

(30) Priorität: **16.11.90 DE 4036647**

(43) Veröffentlichungstag der Anmeldung: **20.05.92 Patentblatt 92/21**

(84) Benannte Vertragsstaaten: **AT BE CH DE DK ES FR GB IT LI NL**

(71) Anmelder: **HOECHST AKTIENGESELLSCHAFT**
**Postfach 80 03 20**
**W-6230 Frankfurt am Main 80(DE)**

(72) Erfinder: **Rupp, Walter, Dr.**
**Am Eichkopf 6**
**W-6240 Königstein/Taunus(DE)**
Erfinder: **Gethöffer, Hanspeter, Dr.**
**Via Alpignano, 120**
**I-10040 Caselette (To)(IT)**
Erfinder: **Reinhardt, Gerd, Dr.**
**Freiherr-v.-Stein-Strasse 37**
**W-6233 Kelkheim (Taunus)(DE)**
Erfinder: **Jaekel, Frank, Dr.**
**Am Carlusbaum 24**
**W-6233 Bad Soden (Taunus)(DE)**

(54) **Sulfimidoperoxicarbonsäuren.**

(57) Sulfimidoperoxicarbonsäuren der Formel

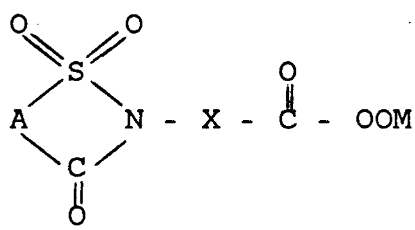

worin
A eine Gruppe der Formel

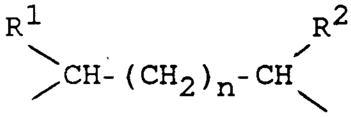 , 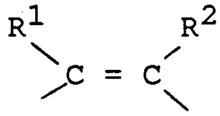 , 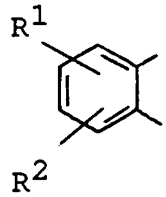 oder

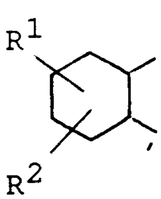

EP 0 485 927 A1

n die Zahlen 0, 1 oder 2,
$R^1$ Wasserstoff, Fluor, Chlor, Brom, $C_1$-$C_{20}$-Alkyl, $C_2$-$C_{20}$-Alkenyl, Aryl oder $C_1$-$C_{10}$-Alkylaryl,
$R^2$ Wasserstoff, Fluor, Chlor, Brom oder eine Gruppe der Formel $SO_3M$, $CO_2M$ oder $OSO_3M$,
M Wasserstoff, ein Alkali- oder Ammonium-Ion oder die stöchiometrische Menge eines Erdalkali-Ions und
X $C_1$-$C_{19}$-Alkylen oder ortho-, meta- oder para-Arylen bedeuten.

Diese Sulfimidoperoxicarbonsäuren eignen sich als Bleich-, Oxidations- oder Desinfektionsmittel.

Die Erfindung bezieht sich auf Sulfimidoperoxicarbonsäuren, Verfahren zu ihrer Herstellung und ihre Verwendung. Anorganische Persalze sind seit langem als Bleichzusätze in Waschmitteln bekannt. Da sie ihre optimale Bleichkraft jedoch erst bei Temperaturen oberhalb 60 ° C entfalten, werden zu ihrer Aktivierung eine Reihe von organischen Verbindungen beschrieben, die während des Waschprozesses mit Wasserstoffperoxid unter Freisetzung einer Peroxicarbonsäure reagieren, welche bereits bei 40 bis 60 ° C bleichend wirkt. Eine Übersicht zahlreicher bekannter Perborataktivatoren wie N-Acylverbindungen (Tetraacetylethylendiamin, Tetraacetylmethylendiamin, Tetraacetylglycoluril) oder aktivierte Ester (Pentaacetylglucose, Acetoxibenzolsulfonat-Natrium, Benzoyloxibenzolsulfonat-Natrium) ist z.B. in der US-Patentschrift 4,248,928 gegeben.

Daneben werden in neuerer Zeit eine Reihe organischer Peroxicarbonsäuren als Bleichsysteme für Waschmittel beschrieben. Neben bereits kommerziell erhältlichen Peroxicarbonsäuren wie Dodecandiperoxicarbonsäure (EP-A-127 782) und Monoperoxiphthalsäure (EP-A-27 693) sind Perbernsteinsäure (DE-A-34 38 529), Perglutarsäure (DE-A-35 39 036), und Sulfoperbenzoesäure beschrieben. Problematisch an diesen Peroxicarbonsäuren ist jedoch ihre geringe Lagerstabilität, die zum Teil erst durch besondere physikalische oder chemische Stabilisierung gewährleistet wird. Insbesondere hat sich hier die Herstellung von Magnesium-Salzen (EP-A-105 689) oder ein Zusatz von Phosphanoxid/Natriumsulfat (DE-A-33 20 497) bewährt. Nach EP-A-170 386 können organische Peroxicarbonsäuren auch durch eine zusätzliche Amidgruppe, nach EP-A-349 940 auch durch eine zusätzliche Imidgruppe im Molekül, stabilisiert werden.

Darüberhinaus werden zahlreiche weitere Peroxicarbonsäuren, die stabilisierende funktionelle Gruppen besitzen, beschrieben, so z.B. Ammoniumperoxicarbonsäuren (EP-A-316 809) Pyridin-N-oxidperoxicarbonsäuren (EP-A-300 461) oder Sulfonperoxicarbonsäuren (EP-A-267 175).

Gegenstand der vorliegenden Erfindung sind Sulfimidoperoxicarbonsäuren der allgemeinen Formel

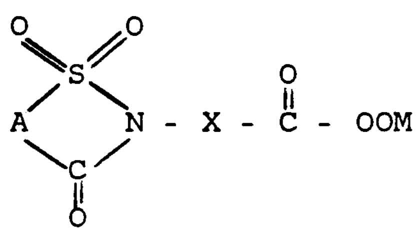

wobei A eine Gruppe der Formel

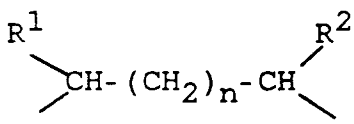 , 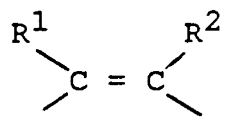 , 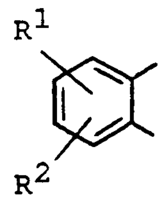 , 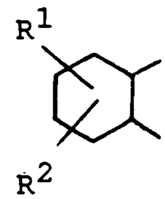

n die Zahlen 0, 1 oder 2

$R^1$ Wasserstoff, Fluor, Chlor, Brom, $C_1$-$C_{20}$-Alkyl, $C_2$-$C_{20}$-Alkenyl, Aryl, bevorzugt Phenyl oder $C_1$-$C_{10}$-Alkylaryl, bevorzugt $C_1$-$C_4$-Alkylphenyl,

$R^2$ Wasserstoff, Fluor, Chlor, Brom oder eine Gruppe der Formel $SO_3M$, $CO_2M$ oder $OSO_3M$

M Wasserstoff, ein Alkali- oder Ammonium-Ion oder die stöchiometrische Menge eines Erdalkali-Ions und

X $C_1$-$C_{19}$-Alkylen oder ortho-, meta- oder para-Arylen, bevorzugt para-Phenylen, bedeuten.

Besonders geeignet für den erfindungsgemäßen Zweck sind Saccharinpercarbonsäuren, die sich in ihrer Alkylenkette unterscheiden, wie z.B. 4-[1,1,3-Trioxo-3H-λ$^6$-benz[d]-isothiazol-2-yl]-perbutansäure, 6-[1,1,3-Trioxo-3H-λ$^6$-benz[d]isothiazol-2-yl]perhexansäure und 7-[1,1,3-Trioxo-3H-λ$^6$-benz[d]isothiazol-2-yl]-perheptansäure.

Die Herstellung der Saccharinpercarbonsäuren erfolgt durch die Schritte

-a- Veresterung der Bromcarbonsäure

-b- Synthese des Saccharincarbonsäureesters

-c- Oxidation zur Saccharinpercarbonsäure

-d- Isolierung der Saccharinpercarbonsäure

Im folgenden werden die einzelnen Schritte näher erläutert. Die Darstellung des Bromcarbonsäureesters Br-X-$COOR^3$, wobei $R^3$ $C_1$-$C_5$-Alkyl bedeutet, erfolgt durch säurekatalysierte Veresterung der Bromcarbonsäure (Houben-Weyl, Methoden der Organischen Chemie, E5, S.65).

Als Bromcarbonsäuren können insbesondere Bromessigsäure, 3-Brompropionsäure, 4-Brombuttersäure, 6-Bromcapronsäure und 7-Bromheptansäure eingesetzt werden.

Der Saccharincarbonsäureester wird durch Umsetzung von Saccharin-Natriumsalz (US-A-1,601,505, US-A-2,667,503) mit dem Bromcarbonsäureester Br-X-$COOR^3$ in Dimethylformamid (J. Org. Chem. 21 (1956), 583) oder aus 2-Sulfobenzoesäureanhydrid und einem Aminosäureester der Formel $H_2$N-X-$COOR^3$ erhalten (US-A-2,462,835).

Die Umwandlung der im Schritt -b- erhaltenen Sulfimidocarbonsäureester zu Sulfimidopercarbonsäuren erfolgt durch Umsetzung mit einem Oxidationsgemisch aus Wasserstoffperoxid und einer starken Säure. Wasserstoffperoxid kommt gewöhnlich als 30 bis 95 gew.-%ige, vorzugsweise 50 bis 85 gew.-%ige, wäßrige Lösung zum Einsatz.

Geeignete saure Katalysatoren sind Schwefelsäure, Methansulfonsäure oder ein saurer Ionenaustauscher. Schwefelsäure wird als 50 bis 96 gew.-%ige, vorzugsweise 75 bis 96 gew.-%ige, wäßrige Lösung verwendet.

Wasserstoffperoxid wird pro oxidierbarer Carboxylgruppe des Sulfimidocarbonsäureesters im molaren Verhältnis 10 bis 1 zu 1, vorzugsweise 4 bis 2 zu 1, eingesetzt. Art und Menge der Katalysatorsäure sind abhängig von dem eingesetzten Sulfimidocarbonsäureester. Im allgemeinen wird die 1.5 bis 6-fache Gewichtsmenge - bezogen auf den Sulfimidocarbonsäureester - an Katalysatorsäure zugesetzt. Die Reaktionstemperatur richtet sich nach der Stabilität der entsprechenden Sulfimidopercarbonsäure und liegt gewöhnlich zwischen 5 und 60 ° C, vorzugsweise 30 bis 45 ° C.

Die beanspruchten Sulfimidoperoxicarbonsäuren fallen im allgemeinen durch Zugabe von Wasser aus und können in einfacher Weise durch Filtration oder Zentrifugation isoliert werden. Es ist auch möglich, Sulfimidoperoxicarbonsäuren, die durch Wasserzugabe nicht oder nur unvollständig ausfallen, durch Zugabe von wäßrigen Lösungen von basischen Salzen auszufällen.

Die erfindungsgemäßen Sulfimidoperoxicarbonsäuren sind fest, nahezu geruchlos, besitzen einen niedrigen Dampfdruck und sind von ausgezeichneter thermischer Stabilität. Sie können als Bleichmittel für Textilien in reiner Form, vorzugsweise als Fleckensalz, oder in sauren Flüssigformulierungen mit einem pH-Wert kleiner oder gleich 6 eingesetzt oder in Formulierungen mit Waschmitteln, vorzugsweise als Granulat, zum Bleichen von Textilien verwendet werden.

Weiterhin können die erfindungsgemäßen Sulfimidoperoxicarbonsäuren als Sanitärreiniger oder als Desinfektionsmittel für medizinische Geräte verwendet werden. Die erfindungsgemäßen Sulfimidoperoxicarbonsäuren eignen sich ferner als Oxidationsmittel in der Synthesechemie, insbesondere für die Epoxidierung von Olefinen.

Die Herstellung der erfindungsgemäßen Sulfinimidoperoxicarbonsäuren wird durch die folgenden Beispiele erläutert:

Beispiel 1

a) 2-[1,1,3-Trioxo-3H-$\lambda^6$-benz[d]isothiazol-2-yl]-essigsäuremethylester.

37,8 g (0,2 Mol) wasserfreies Saccharin-Natriumsalz werden in 60 ml wasserfreiem Dimethylformamid (DMF) vorgelegt und mit einer Lösung von 30,3 g (0,2 Mol) $\alpha$-Bromessigsäuremethylester in 40 ml wasserfreiem DMF versetzt. Anschließend wird 5 h bei 100 ° C gerührt und nach dem Abkühlen 100 ml Wasser zugetropft. Das Reaktionsprodukt wird dreimal mit je 100 ml Chloroform aus der wäßrigen Phase ausgeschüttelt, die organische Phase über Natriumsulfat getrocknet und die Lösungsmittel am Rotationsverdampfer abgezogen.

Ausbeute 46,6 g (97,5 %) Fp.: 104-106 ° C

$^1$H-NMR ($CDCl_3$, 100 MHz):$\delta$ 3,8 (s,3H), 4,45 (s,2H), 7,8-8,15 (m,4H)

b) 2-[1,1,3-Trioxo-3H-$\lambda^6$-benz[d]isothiazol-2-yl]-peressigsäure.

23,9 g (0,1 Mol) 2-[1,1,3-Trioxo-3H-$\lambda^6$-benz[d]-isothiazol-2-yl-essigsäuremethylester werden in 50 g Schwefelsäure (96 gew.-%ig) gelöst und die Lösung auf 40 ° C abgekühlt. Unter Eiskühlung werden dann 10 g (0,25 Mol) Wasserstoffperoxid (85 gew.-%ig) so zugetropft, daß die Innentemperatur zwischen 35 und 40 ° C gehalten werden kann. Danach werden unter Kühlung 100 ml Wasser zugetropft, die Reaktionsmischung fünfmal mit je 150 ml Methylenchlorid ausgeschüttelt, die organische Phase viermal mit je 200 ml Wasser neutral gewaschen und über $Na_2SO_4$ getrocknet. Das Lösungsmittel wird am Rotationsverdampfer bei einer Wasserbadtemperatur von maximal 40 °C abgezogen und das Produkt bei 40 °C im Wasserstrahlvakuum getrocknet.
Ausbeute: 15,7 g (65 %) Aktivsauerstoffgehalt (OA) = 3,5 %
Wirksubstanzgehalt (WS) = 53,7 %
Fp.: 100 °C (Zersetzung)
$^1$H-NMR ($CDCl_3$, 100 MHz):$\delta$ 4,59 (s,2H), 7,8-8,15 (m,4H)

Beispiel 2

a) 3-[1,1,3-Trioxo-3H-$\lambda^6$-benz[d]isothiazol-2-yl]-propionsäuremethylester.

37,8 g (0,2 Mol) wasserfreies Saccharin-Natriumsalz in 60 ml wasserfreiem DMF und 33,4 g (0,2 Mol) $\beta$-Brompropionsäuremethylester werden wie in Beispiel 1 beschrieben zur Reaktion gebracht und aufgearbeitet.
Ausbeute: 47,4 g (94 %) Fp.: 63-64 °C
$^1$H-NMR ($CDCl_3$, 100 MHz):$\delta$ 2,88 (t,2H), 3,74 (s,3H), 4,1 (t,2H), 7,8-8,13 (m)

b) 3-[1,1,3-Trioxo-3H-$\lambda^6$-benz[d]isothiazol-2-yl]-perpropionsäure.

25,3 g (0,1 Mol) 3-[1,1,3-Trioxo-3H-$\lambda^6$-benz[d]isothiazol-2-yl]-propionsäuremethylester werden in 50 g Schwefelsäure (96 gew.-%ig) gelöst und die Lösung auf 35 °C abgekühlt. Unter Eiskühlung werden 17 g (0,25 Mol) Wasserstoffperoxid (50 gew.-%ig) so zugetropft, daß die Innentemperatur zwischen 35-40 °C gehalten werden kann. Nach 1h Rühren bei 40 °C werden noch etwa 100 ml Wasser unter Kühlung zugegeben. Die ausgefallene Peroxicarbonsäure wird abgesaugt, mit Wasser mineralsäurefrei gewaschen und bei 40 °C im Wasserstrahlvakuum getrocknet.
Ausbeute: 15,9 g (62,4 %), OA = 4,27 %, WS = 68 %
Fp.: 101-102 °C (Zersetzung)
$^1$H-NMR ($CDCl_3$, 100 MHz):$\delta$ 2,98 (t,2H), 4,15 (t,2H), 7,8-8,13 (m).

Beispiel 3

a) 4-[1,1,3-Trioxo-3H-$\lambda^6$-benz[d]isothiazol-2-yl]-butansäuremethylester.

120 g (0,64 Mol) wasserfreies Saccharin-Natriumsalz in 200 ml wasserfreiem DMF und 115 g (0,64 Mol) $\gamma$-Brombutansäuremethylester in 115 ml wasserfreiem DMF werden wie in Beispiel 1 zur Reaktion gebracht und aufgearbeitet.
Ausbeute: 168,4 g (98,5 %) Fp.: 91-92 °C
$^1$H-NMR ($CDCl_3$, 100 MHz):$\delta$ 2,18 (m,2H), 2,5 (m,2H), 3,7 (s,3H), 3,88 (t,2H), 7,8-8,13 (m,4H)

b) 4-[1,1,3-Trioxo-3H-$\lambda^6$-benz[d]isothiazol-2-yl]-perbutansäure.

26,7 g (0,1 Mol) 4-[1,1,3-Trioxo-3H-$\lambda^6$-benz[d]isothiazol-2-yl]-butansäuremethylester, 50 g Schwefelsäure (96 gew.%ig) und 17 g (0,25 Mol) Wasserstoffperoxid (50 gew.-%ig) werden wie in Beispiel 2 zur Reaktion gebracht, 3 h bei 40 °C gerührt und wie in Beispiel 2 aufgearbeitet.
Ausbeute: 24,6 g (91,6 %), OA = 4,36 %, WS = 73,4 %
Fp.: 47-51 °C

Beispiel 4

a) 6-[1,1,3-Trioxo-3H-$\lambda^6$-benz[d]isothiazol-2-yl]-hexansäuremethylester.

167,4 g (0,89 Mol) wasserfreies Saccharin-Natriumsalz in 240 ml wasserfreiem DMF und 185 g (0,89 Mol) $\epsilon$-Bromcapronsäuremethylester in 160 ml wasserfreiem DMF werden wie in Beispiel 1 beschrieben zur Reaktion gebracht und aufgearbeitet.
Ausbeute: 267,4 g (97 %) Fp.: 82-84 °C

b) 6-[1,1,3-Trioxo-3H-$\lambda^6$-benz[d]isothiazol-2-yl]-perhexansäure.

29,5 g (0,1 Mol) 6-[1,1,3-Trioxo-3H-$\lambda^6$-benz[d]isothiazol-2-yl]-hexansäuremethylester, 50 g Schwefelsäure (96 gew.-%ig) und 17 g (0,25 Mol) Wasserstoffperoxid (50 gew.-%ig) werden wie in Beispiel 2 zur Reaktion gebracht, 2 h bei 40 °C nachgerührt und aufgearbeitet.
Ausbeute: 28,7 g (96,6 %), OA = 4,2 %, WS = 75,3 %
Fp.: wachsartig
$^1$H-NMR ($CDCl_3$, 100 MHz):$\delta$ 1,3-2,0 (m,6H), 2,4 (m,2H), 3,8 (m,2H), 7,8-8,1 (m,4H)

Beispiel 5

a) 7-[1,1,3-Trioxo-3H-$\lambda^6$-benz[d]isothiazol-2-yl]-heptansäuremethylester.

238 g (1,26 Mol) wasserfreies Saccharin-Natriumsalz in 450 ml wasserfreiem DMF und 282 g (1,26 Mol) 7-Bromheptansäuremethylester in 280 ml wasserfreiem DMF werden wie in Beispiel 1 beschrieben zur Reaktion gebracht und aufgearbeitet.
Ausbeute: 394 g (96 %) Fp.: 62-63 °C
$^1$H-NMR ($CDCl_3$, 100 MHz):$\delta$ 1,25-2,0 (bm,8H), 2,3 (t,2H), 3,65 (s,3H), 3,75 (t,2H), 7,75-8,1 (m,4H)

b) 7-[1,1,3-Trioxo-3H-$\lambda^6$-benz[d]isothiazol-2-yl]-perheptansäure.

30 g (0,1 Mol) 7-[1,1,3-Trioxo-3H-$\lambda^6$-benz[d]isothiazol-2-yl]-heptansäuremethylester, 50 g Schwefelsäure (96 gew.-%ig) und 17 g (0,25 Mol) Wasserstoffperoxid (50 gew.-%ig) werden wie in Beispiel 2 zur Reaktion gebracht, 2 h zwischen 40 und 43 °C gerührt und aufgearbeitet.
Ausbeute: 28,8 g (92,6 %), OA = 4,44 %, WS = 86,4 %
Fp.: 63-64 °C

Waschversuche im Launder-o-meter

Die Waschversuche werden im vorgeheizten Launder-o-meter bei Temperaturen von 20, 40 und 60 °C unter Verwendung von Wasser der Wasserhärte 15 °dH durchgeführt. Die Waschzeit beträgt jeweils 30 min.

Als Testwaschmittel werden 1,5 g/l phosphatfreies WMP-Waschmittel (Wäschereiforschung Krefeld) zugesetzt. Die in pulverisierter Form vorliegenden Persäuren werden so dosiert, daß sie bei vollständiger Auflösung jeweils 25 mg/l Aktivsauerstoff freisetzen können.

Als Standardanschmutzungen dienen Tee auf Baumwolle (Wäschereiforschung Krefeld) und Rotwein auf Baumwolle (EMPA, Schweiz). Die Bleichleistung ist als Remissionswert nach der Wäsche, gemessen bei 460 nm, angegeben.

Als Vergleichssubstanz wird Tetraacetylethylendiamin (TAED) in Kombination mit Perborat-Monohydrat eingesetzt.

| Remissionswerte | Tee / Baumwolle | | |
|---|---|---|---|
| Verbindung | 20°C | 40°C | 60°C |
| gemäß Beispiel 1 | 62,9 | - | - |
| gemäß Beispiel 2 | 63,8 | - | - |
| gemäß Beispiel 3 | 62,1 | 71,8 | 74,6 |
| gemäß Beispiel 4 | 61,1 | 69,4 | 74,2 |
| TAED/Perborat | 58,1 | 66,4 | 72,0 |

| Remissionswerte | Rotwein / Baumwolle | | |
|---|---|---|---|
| Verbindung | 20°C | 40°C | 60°C |
| gemäß Beispiel 1 | 65,7 | - | - |
| gemäß Beispiel 2 | 63,4 | - | - |
| gemäß Beispiel 3 | 62,2 | 71,6 | 76,9 |
| gemäß Beispiel 4 | 62,1 | 70,4 | 75,7 |
| TAED/Perborat | 57,9 | 65,9 | 68,2 |

Die Waschversuche zeigen, daß die erfindungsgemäßen Sulfimidoperoxicarbonsäuren bei 20 ° C, 40 ° C und 60 ° C höhere Remissionswerte und somit eine bessere Bleichleistung aufweisen als die Vergleichssubstanz TAED/Perborat.

**Patentansprüche**

1. Sulfimidoperoxicarbonsäuren der Formel

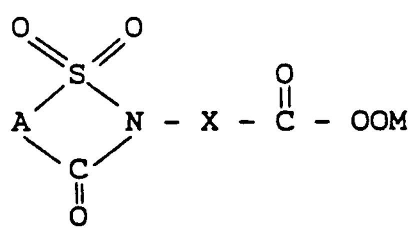

worin
A eine Gruppe der Formel

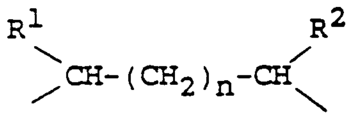,
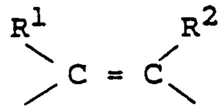,
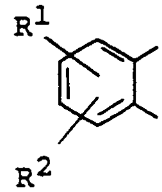 oder

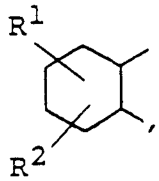,

n die Zahlen 0, 1 oder 2,
$R^1$ Wasserstoff, Fluor, Chlor, Brom, $C_1$-$C_{20}$-Alkyl, $C_2$-$C_{20}$-Alkenyl, Aryl oder $C_1$-$C_{10}$-Alkylaryl,
$R^2$ Wasserstoff, Fluor, Chlor, Brom oder eine Gruppe der Formel $SO_3M$, $CO_2M$ oder $OSO_3M$,
M Wasserstoff, ein Alkali- oder Ammonium-Ion oder die stöchiometrische Menge eines Erdalkali-Ions und
X $C_1$-$C_{19}$-Alkylen oder ortho-, meta- oder para-Arylen bedeuten.

**2.** Verbindungen nach Anspruch 1, worin A eine Gruppe der Formel

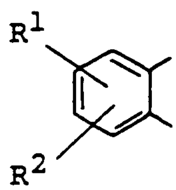

worin
$R^1$ Wasserstoff, Fluor, Chlor, Brom, Phenyl oder $C_1$-$C_4$-Alkylphenyl,
$R^2$ Wasserstoff, Fluor, Chlor, Brom oder eine Gruppe der Formel $SO_3M$, COOM oder $OSO_3M$,
M Wasserstoff, ein Alkali- oder Ammonium-Ion oder die stöchiometrische Menge eines Erdalkali-Ions und
X $C_1$-$C_{19}$-Alkylen oder para-Phenylen bedeuten.

**3.** Verbindungen nach Anspruch 2, worin A, $R^1$, $R^2$ und M die dort beschriebene Bedeutung haben und X $C_1$-$C_6$-Alkylen bedeutet.

**4.** Saccharinperoxicarbonsäuren nach Anspruch 3, worin A, M und X die dort beschriebene Bedeutung haben und $R^1$ und $R^2$ jeweils Wasserstoff bedeuten.

**5.** Verfahren zur Herstellung der Verbindungen nach mindestens einem der Ansprüche 1 bis 4, dadurch gekennzeichnet, daß ein Sulfimidocarbonsäureester der Formel

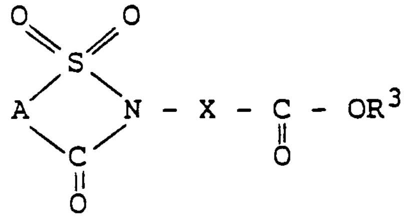

worin
A eine Gruppe der Formel

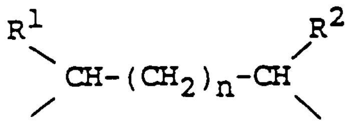 , 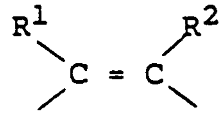 , 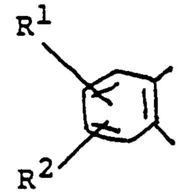 oder

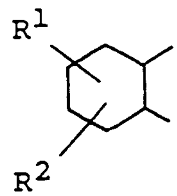 ,

n die Zahlen 0, 1 oder 2,

$R^1$ Wasserstoff, Fluor, Chlor, Brom, $C_1$-$C_{20}$-Alkyl, $C_2$-$C_{20}$-Alkenyl, Aryl oder $C_1$-$C_{10}$-Alkylaryl,

$R^2$ Wasserstoff, Fluor, Chlor, Brom oder eine Gruppe der Formel $SO_3M$, $CO_2M$ oder $OSO_3M$,

M Wasserstoff, ein Alkali- oder Ammonium-Ion oder die stöchiometrische Menge eines Erdalkali-Ions und

X $C_1$-$C_{19}$-Alkylen oder ortho-, meta- oder para-Arylen

$R^3$ $C_1$-$C_5$-Alkyl

bedeuten, mit einem Gemisch aus wäßrigem Wasserstoffperoxid und einer starken Säure, vorzugsweise Schwefelsäure, Methansulfonsäure oder ein saurer Ionenaustauscher, umgesetzt wird, wobei Wasserstoffperoxid im molaren Verhältnis 10 bis 1 zu 1, vorzugsweise 4 bis 2 zu 1, pro oxidierbarer Carboxylgruppe des Sulfimidocarbonsäureesters und die 1,5 bis 6-fache Gewichtsmenge der starken Säure, bezogen auf den Sulfimidocarbonsäureester, eingesetzt wird.

**6.** Verfahren nach Anspruch 5, dadurch gekennzeichnet, daß eine 30 bis 95 gew.-%ige wäßrige Wasserstoffperoxid-Lösung verwendet wird.

**7.** Verfahren gemäß Anspruch 5 oder 6, dadurch gekennzeichnet, daß als starke Säure 50 bis 96 gew.-%ige, vorzugsweise 75 bis 96 gew.-%ige, Schwefelsäure verwendet wird.

**8.** Verfahren gemäß einem der Ansprüche 5 bis 7, dadurch gekennzeichnet, daß die Reaktionstemperatur zwischen 5 und 60, insbesondere 30 und 45 ° C, liegt.

**9.** Verwendung der Verbindungen gemäß einem der Ansprüche 1 bis 4 als Bleich-, Oxidations- oder Desinfektionsmittel.

**Patentansprüche für folgenden Vertragsstaat : ES**

**1.** Bleich-, Oxidations- oder Desinfektionsmittel enthaltend Sulfimidoperoxicarbonsäuren der Formel

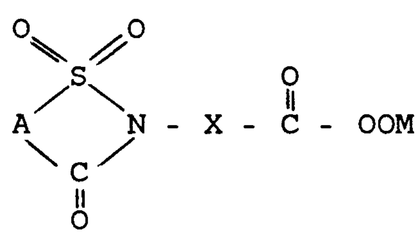

worin

A eine Gruppe der Formel

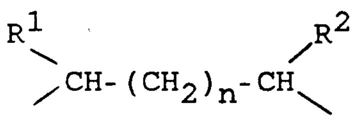 , 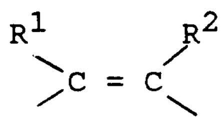 , 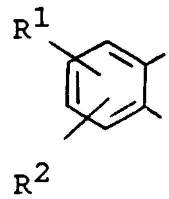 oder

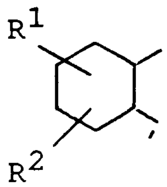 ,

n die Zahlen 0, 1 oder 2,
$R^1$ Wasserstoff, Fluor, Chlor, Brom, $C_1$-$C_{20}$-Alkyl, $C_2$-$C_{20}$-Alkenyl, Aryl oder $C_1$-$C_{10}$-Alkylaryl,
$R^2$ Wasserstoff, Fluor, Chlor, Brom oder eine Gruppe der Formel $SO_3M$, $CO_2M$ oder $OSO_3M$,
M Wasserstoff, ein Alkali- oder Ammonium-Ion oder die stöchiometrische Menge eines Erdalkali-Ions und
X $C_1$-$C_{19}$-Alkylen oder ortho-, meta- oder para-Arylen bedeuten.

2. Bleich-, Oxidations- oder Desinfektionsmittel enthaltend Verbindungen nach Anspruch 1, worin A eine Gruppe der Formel

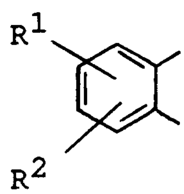

worin
$R^1$ Wasserstoff, Fluor, Chlor, Brom, Phenyl oder $C_1$-$C_4$-Alkylphenyl,
$R^2$ Wasserstoff, Fluor, Chlor, Brom oder eine Gruppe der Formel $SO_3M$, COOM oder $OSO_3M$,
M Wasserstoff, ein Alkali- oder Ammonium-Ion oder die stöchiometrische Menge eines Erdalkali-Ions und
X $C_1$-$C_{19}$-Alkylen oder para-Phenylen bedeuten.

3. Bleich-, Oxidations- oder Desinfektionsmittel enthaltend Verbindungen nach Anspruch 2, worin A, $R^1$, $R^2$ und M die dort beschriebene Bedeutung haben und X $C_1$-$C_6$-Alkylen bedeutet.

4. Bleich-, Oxidations- oder Desinfektionsmittel enthaltend Saccharinperoxicarbonsäuren nach Anspruch 3, worin A, M und X die dort beschriebene Bedeutung haben und $R^1$ und $R^2$ jeweils Wasserstoff bedeuten.

5. Verfahren zur Herstellung der Verbindungen nach mindestens einem der Ansprüche 1 bis 4, dadurch gekennzeichnet, daß ein Sulfimidocarbonsäureester der Formel

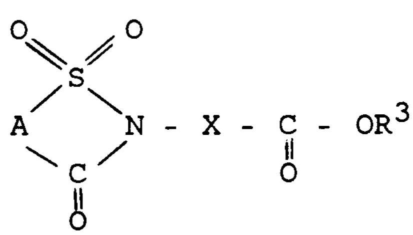

worin

A eine Gruppe der Formel

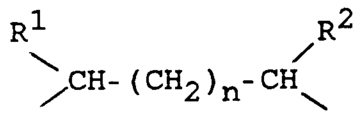 , 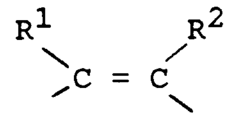 , 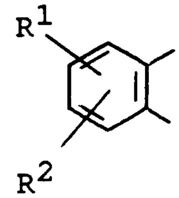 oder

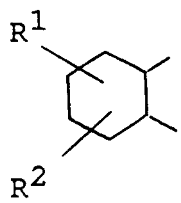 ,

n die Zahlen 0, 1 oder 2,

$R^1$ Wasserstoff, Fluor, Chlor, Brom, $C_1$-$C_{20}$-Alkyl, $C_2$-$C_{20}$-Alkenyl, Aryl oder $C_1$-$C_{10}$-Alkylaryl,

$R^2$ Wasserstoff, Fluor, Chlor, Brom oder eine Gruppe der Formel $SO_3M$, $CO_2M$ oder $OSO_3M$,

M Wasserstoff, ein Alkali- oder Ammonium-Ion oder die stöchiometrische Menge eines Erdalkali-Ions und

X $C_1$-$C_{19}$-Alkylen oder ortho-, meta- oder para-Arylen

$R^3$ $C_1$-$C_5$-Alkyl

bedeuten, mit einem Gemisch aus wäßrigem Wasserstoffperoxid und einer starken Säure, vorzugsweise Schwefelsäure, Methansulfonsäure oder ein saurer Ionenaustauscher, umgesetzt wird, wobei Wasserstoffperoxid im molaren Verhältnis 10 bis 1 zu 1, vorzugsweise 4 bis 2 zu 1, pro oxidierbarer Carboxylgruppe des Sulfimidocarbonsäureesters und die 1,5 bis 6-fache Gewichtsmenge der starken Säure, bezogen auf den Sulfimidocarbonsäureester, eingesetzt wird.

**6.** Verfahren nach Anspruch 5, dadurch gekennzeichnet, daß eine 30 bis 95 gew.-%ige wäßrige Wasserstoffperoxid-Lösung verwendet wird.

**7.** Verfahren gemäß Anspruch 5 oder 6, dadurch gekennzeichnet, daß als starke Säure 50 bis 96 gew.-%ige, vorzugsweise 75 bis 96 gew.-%ige, Schwefelsäure verwendet wird.

**8.** Verfahren gemäß einem der Ansprüche 5 bis 7, dadurch gekennzeichnet, daß die Reaktionstemperatur zwischen 5 und 60, insbesondere 30 und 45 ° C, liegt.

Europäisches Patentamt

# EUROPÄISCHER RECHERCHENBERICHT

Nummer der Anmeldung

EP 91 11 9184

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A,D | EP-A-0 349 940 (HOECHST AKTIENGESELLSCHAFT)<br>* Ansprüche * | 1,9 | C07D275/06<br>C11D3/395<br>C07D275/02 |
| D,A | EP-A-0 316 809 (AUSIMONT S.R.I.)<br>* Ansprüche 1,8,18 * | 1,9 | |
| D,A | EP-A-0 267 175 (MONSANTO COMPANY)<br>* Ansprüche * | 1,9 | |
| | | | RECHERCHIERTE SACHGEBIETE (Int. Cl.5 ) |
| | | | C07D<br>C11D |

Der vorliegende Recherchenbericht wurde für alle Patentansprüche erstellt

| Recherchenort | Abschlußdatum der Recherche | Prüfer |
|---|---|---|
| DEN HAAG | 03 FEBRUAR 1992 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)